# EUROPEAN PATENT SPECIFICATION

(11) **EP 4 508 186 B1**
(45) Date of publication and mention of the grant of the patent: **11.03.2026**
(21) Application number: 23719359.4
(22) Date of filing: 12.04.2023
(51) Int. Cl.: C12M 1/12, C12M 1/34, C12Q 1/02, G01N 33/50

(54) **SENSOR DEVICE FOR IN-VITRO AEROSOL EXPOSURE**
SENSORVORRICHTUNG FÜR IN-VITRO-AEROSOLEXPOSITION
DISPOSITIF DE CAPTEUR D'EXPOSITION D'AÉROSOL IN VITRO

(30) Priority: 12.04.2022 EP 22167993
(43) Date of publication of application: 19.02.2025
(73) Proprietor: Philip Morris Products S.A., 2000 Neuchâtel (CH)
(72) Inventor: STEINER, Sandro, 2000 Neuchâtel (CH)
(74) Representative: Maiwald GmbH
(86) International application number: PCT/EP2023/059562
(87) International publication number: WO 2023/198776

(56) References cited:
- JASON ADAMSON ET AL: "Real-time assessment of cigarette smoke particle deposition in vitro", CHEMISTRY CENTRAL JOURNAL, BIOMED CENTRAL LTD, LO, vol. 6, no. 1, 10 September 2012 (2012-09-10), pages 98, XP021116970, ISSN: 1752-153X, DOI: 10.1186/1752-153X-6-98
- JASON ADAMSON ET AL: "Assessment of cigarette smoke particle deposition within the Vitrocell exposure module using quartz crystal microbalances", CHEMISTRY CENTRAL JOURNAL, BIOMED CENTRAL LTD, LO, vol. 7, no. 1, 12 March 2013 (2013-03-12), pages 50, XP021145808, ISSN: 1752-153X, DOI: 10.1186/1752-153X-7-50
- DAVID TOTH ET AL: "High-sensitivity electrochemical dual-QCM for reliable three-electrode measurements", ARXIV.ORG, CORNELL UNIVERSITY LIBRARY, 201 OLIN LIBRARY CORNELL UNIVERSITY ITHACA, NY 14853, 7 March 2021 (2021-03-07), XP081907326
- SECONDO LYNN E. ET AL: "Real-time monitoring of cellular oxidative stress during aerosol sampling: a proof of concept study", DRUG AND CHEMICAL TOXICOLOGY., vol. 45, no. 2, 4 March 2022 (2022-03-04), US, pages 767 - 774, XP093067012, ISSN: 0148-0545, Retrieved from the Internet <URL:https://www.ncbi.nlm.nih.gov/pmc/articles/PMC7736142/pdf/nihms-1642751.pdf> DOI: 10.1080/01480545.2020.1774774
- ADEEL AFZAL ET AL: "Gravimetric Viral Diagnostics: QCM Based Biosensors for Early Detection of Viruses", CHEMOSENSORS, vol. 5, no. 1, 13 February 2017 (2017-02-13), pages 7, XP055744762, DOI: 10.3390/chemosensors5010007
- RAMIAH RAJASEKARAN PRADEEP ET AL: "3D-Printed electrochemical sensor-integrated transwell systems", vol. 6, no. 1, 1 December 2020 (2020-12-01), pages 100, XP055922863, Retrieved from the Internet <URL:https://www.nature.com/articles/s41378-020-00208-z.pdf> DOI: 10.1038/s41378-020-00208-z
- FUCHS STEFANIE ET AL: "In-Line Analysis of Organ-on-Chip Systems with Sensors: Integration, Fabrication, Challenges, and Potential", vol. 7, no. 7, 12 July 2021 (2021-07-12), pages 2926 - 2948, XP055964376, ISSN: 2373-9878, Retrieved from the Internet <URL:https://pubs.acs.org/doi/pdf/10.1021/acsbiomaterials.0c01110> DOI: 10.1021/acsbiomaterials.0c01110
- SAYES CHRISTIE M ET AL: "The link between delivered aerosol dose and inflammatory responses: Exposing a lung Cell Co-Culture system to selected Allergens and irritants", JOURNAL OF AEROSOL SCIENCE, ELSEVIER, AMSTERDAM, NL, vol. 151, 22 September 2020 (2020-09-22), XP086349531, ISSN: 0021-8502, [retrieved on 20200922], DOI: 10.1016/J.JAEROSCI.2020.105677

## Description

The present disclosure generally relates to sensor devices and systems for in-vitro exposure systems, in particular in-vitro aerosol exposure systems. Specifically, the present disclosure relates to a sensor device for an aerosol exposure system for in-vitro exposure of a cell culture or a surrogate thereof to a test atmosphere. Further, the present disclosure relates to an aerosol exposure system comprising one or more of such sensor devices, to a use of such sensor device or exposure system, and to a method of determining in-vitro exposure of a surrogate for a cell culture.

The development and safety assessment of products, for example consumer products, containing one or more inhalable substances that can be inhaled intentionally, as is for example the case with aerosol-generating devices, vaporizers, electronic cigarettes or inhalable drugs, or unintentionally, as is for example the case with air fresheners or deodorant sprays, and the toxicological assessment of inhaled environmental and occupational agents, such as for example combustion engine emissions, spray paint or kitchen fumes, increasingly employ in vitro methodologies using in-vitro exposure systems. Typically, these methodologies rely on the exposure of cell cultures of certain cells, for example cells of the human respiratory tract epithelia, to the substance under investigation. The actual exposures are usually followed by downstream biological or biochemical analyses of, for instance, cellular integrity, transcriptional activity, ciliary beating, oxidative stress or DNA adduct formation to assess a safety or risk of the substance or constituent under investigation.

The substance under investigation may be referred to herein as test atmosphere containing one or more constituents. The test atmosphere or substance under investigation may be or comprise a gas, a mixture of gases, or a suspension of liquid or solid particles in a gaseous matrix, which are generally referred to as aerosols or aerosol particles. Any gaseous or solid component of the test atmosphere may be referred to herein as constituent or component of the test atmosphere.

Usually, in-vitro exposures to test atmospheres are performed at the air liquid interface ("ALI"), a configuration in which the cell culture forms a barrier between a liquid or gelatinous compartment through which nutrients and water are supplied and an airspace in which the test atmosphere is supplied. An alternative approach is submerged mode exposure, where a part of the test atmosphere is dissolved in a liquid or gelatinous matrix, for example with which the cell cultures are covered for exposure. This approach may be less suitable for testing gases or aerosols because the process of trapping such constituent of the test atmosphere in liquid or gelatinous matrices may interfere with its physicochemical properties and thereby its bioactivity. Also, the conditions under which interactions between submerged cell cultures and the test atmosphere take place may not be physiologically representative of the typical environment of the cells, such as the respiratory tract, which may further affect the results obtained in submerged exposure experiments or applications.

ALI exposures, experiments or applications, on the other hand, are usually conducted in aerosol exposure systems in which cell cultures can be brought into contact with test atmospheres under controlled conditions. Aerosol exposure systems typically provide exposure chambers in which cell cultures can be placed and through which test atmospheres can be streamed in controlled manner, for example controlled in terms of flow rate and/or composition of the test atmosphere.

In vitro exposures at the ALI are often performed using so-called transwell cell culture inserts, which may vary in geometry, but may generally feature a cylindrical or slightly conical beaker usually made of polystyrene. The bottom of the beaker typically consists of a porous membrane, such as PET membrane. They are available in various standardized size formats matching 24-, 12- and 6-well cell culture plates. For cell culture cultivation, inserts can be placed into individual wells of a cell culture plate of matching format, containing adequate cell culture medium. A cell suspension can be added into each insert, and the cells sediment, proliferate and over time form a confluent epithelial layer. For ALI applications, the culture medium can be removed from the insert, for example once a confluent epithelial layer is obtained. After this switch from the submerged cultivation to the ALI cultivation, the cells are usually in contact with the surrounding air at the apical side or within the beaker, and in contact with the cell culture plate, the pores in the insert porous membrane and the cell culture medium on the basolateral side. For exposures to test atmospheres, the complete inserts are typically transferred into the exposure chamber of an exposure system.

In-vitro exposure experiments or applications usually aim at quantifying a dose associated with the test atmosphere or one or more constituents thereof. This may include, for example, determining a measure correlating with the dose. As used herein, the dose associated with one or more constituents may refer to one or both an administered dose and a delivered dose. Therein, the administered dose may be indicative of a mass of one or more constituents of the test atmosphere supplied to an exposure chamber or one or more cell cultures contained therein. The delivered dose, on the other hand, may be indicative of a mass of one or more constituents of the test atmosphere that is deposited on or that has diffused into the test system during the exposure, for example into a biological test system or the sensor device as described herein.

The administered dose can usually be controlled during exposure experiments, for instance by controlling the volume flow rate of test atmosphere through the exposure chamber and the exposure duration, and can be calculated based on one or more of these parameters and based on the composition of the test atmosphere, which should preferably be known.

The delivered dose may be a fraction of the administered dose and may arise from or be attributed to mass transfer from the administered dose onto and/or into the cell culture. The kinetics of this mass transfer can be complex; they may be a function of various parameters, such as the particle sizes in case of aerosols, the flow velocities, the molecular masses and the volatility of test atmosphere constituents, as well as potentially their solubility in a liquid lining or layer of the cell cultures. The mass transfer kinetics therefore may vary for different test atmospheres and different constituents of a given test atmosphere and may be dependent on the experimental design, the cell cultures themselves, and the functional principle and design of the used exposure system. As a consequence, delivered doses are often not inferable from administered doses and it may be challenging to compute or otherwise determine the delivered dose or another reliable measure correlating therewith with sufficient accuracy and precision.

Biological or biochemical responses to the exposures, which are typically assessed in an in-vitro exposure application or experiment, however, are usually primarily attributed to the delivered dose, as only constituents of the test atmosphere contributing to the delivered dose may chemically and/or physically interact with the cell cultures. Hence, for accurately assessing biological and/or biochemical responses of cells in aerosol exposure applications or experiments, it may be favourable to accurately determine the delivered dose and/or a measure correlating therewith.

Measuring delivered doses during in vitro exposures to inhalable test substances or test atmospheres is commonly achieved by monitoring the test atmosphere deposition throughout the exposure, which is also referred to as online monitoring. In the alternative approach, the so-called post-hoc quantification, the deposited material and/or the mass of the one or more constituents deposited in an exposed liquid or gelatinous matrix or on an exposed support structure may be determined after the actual exposure with the test atmosphere.

Online monitoring typically relies on quartz crystal microbalances ("QCMs"), which can be placed in one or several of the exposure chambers or wells of a multi-well plate instead of cell cultures. Although QCMs are of low costs, simple in operation and of high sensitivity, for example down to nanograms per cm², quantitative measurement of test atmosphere delivery to cell cultures may be challenging. For instance, QCM's may only report the overall mass deposition, which may be a limiting factor in certain applications, for example if a specific constituent of the test atmosphere is to be determined or measured. If all constituents of a test atmosphere, their mass fraction in the test atmosphere and their deposition kinetics are known, the mass deposition of a targeted constituent may be theoretically inferred or computed from the total mass deposition reported by QCMs. In practice, however, the test atmospheres are commonly not characterized in such detail. Highly complex mixtures, as is for example the case with combustion products, may consist of thousands of constituents. Many of them may not be known and their contribution to the total mass of the test atmosphere as well as the rate at which they deposit on QCMs may potentially differ, even by orders of magnitude. Deriving the accurate mass deposition of an individual constituent from the overall mass deposition reported by QCMs in such cases may hardly be possible. In addition, the physical principle of mass detection by QCMs relies on the coupling of oscillations between the quartz crystal and the deposited mass. This coupling depends on the viscoelastic properties of the deposited material and is only quantitative for solid materials. Within liquid layers, however, the oscillation is typically dampened with increasing distance from the QCM surface. As the thickness of the deposited layer increases, additional deposited mass will be detected with decreasing sensitivity, resulting in an underestimation of mass deposition. Moreover, the surface properties of QCMs may not be physically or chemically representative of a cell culture surface. For instance, highly volatile test atmosphere constituents may efficiently diffuse into cell cultures, but if diffused onto the surface of a QCM, they may re-evaporate and will not be detected as a deposited mass. Finally, incorporation of QCMs into existing aerosol exposure systems can be challenging.

When compared to online monitoring, post-hoc determination of the delivered dose or a measure correlating therewith can offer a high level of selectivity and sensitivity. Post-hoc determination may, for example, comprise mass spectrometry coupled with chromatography, photospectrometry or microscopy, which may be employed after exposure of cell cultures or a suitable surrogate thereof with the test atmosphere to accurately determine the actually delivered dose. Microscopy may be used for dose determination if the targeted test atmosphere constituents are solid particles or constituents of solid particles. Depending on the particle size, light microscopy or electron microscopy may be applied, and the deposited dose may be determined, for example, based on the measured particle number per area and the particle size distribution, particle morphology and/or estimates on the particle surface area. Although such kind of post-hoc measurement and analysis may provide high selectivity and sensitivity, it can be time consuming and may rely on the availability of expensive equipment, such as an electron microscope or mass spectrometer, as well as highly specialized staff to operate these devices. Jason Adamson et al., Chemistry Central Journal, vol. 6, no. 1, 2012, describes a device for real-time assessment of cigarette smoke particles using an insert culture well device adapted to receive QCM.

It may therefore be desirable to provide for an improved sensor device for an aerosol exposure system for in-vitro exposure of a cell culture to a test atmosphere and/or to a surrogate of such cell culture.

This is achieved by the subject-matter of the independent claims. Optional features are provided by the dependent claims and by the following description.

Aspects of the present disclosure relate to a sensor device for an in-vitro aerosol exposure system, to an aerosol exposure system with one or more sensor devices, to the use of such sensor device and/or aerosol exposure system, and to a method of determining in-vitro exposure of a surrogate for a cell culture to a test atmosphere. Any disclosure presented hereinabove and hereinbelow with reference to one aspect of the present disclosure, equally applies to any other aspect of the present disclosure.

According to an aspect of the present disclosure, there is provided a sensor device for an aerosol exposure system for in-vitro exposure of a cell culture and/or a surrogate for a cell culture to a test atmosphere. Therein, the surrogate may refer to a structure or configuration mimicking, replicating and/or resembling a cell culture or surface thereof. The sensor device comprises a measurement compartment configured to receive a receptor element of an electrochemical sensor for detecting one or more constituents of the test atmosphere. The sensor device further includes a receptor support arranged at an end of the measurement compartment and configured to support, carry and/or hold the receptor element of the electrochemical sensor. The sensor device further includes a transducer element functionally couplable to the receptor element and configured to provide an electronically processable signal indicative of the one or more constituents in the test atmosphere.

The sensor device according to the present disclosure may refer to or denote a structure carrying one or more transducer elements that can be coupled with one or more receptor elements of one or more electrochemical sensors. It is noted that any reference to a single transducer element, receptor element and/or electrochemical sensor hereinabove and hereinbelow includes a plurality of transducer elements, receptor elements and/or electrochemical sensors.

The sensor device according to the present disclosure can, for example, be inserted into an aerosol exposure system and/or into an exposure chamber of an exposure system, for example to determine and/or measure the exposure of the sensor device to the one or more constituents of the test atmosphere. Optionally, the sensor device may be configured to quantify the exposure of the sensor device to the one or more constituents of the test atmosphere, for example based on determining a dose or measure correlating with the dose associated with the one or more constituents of the test atmosphere.

The sensor device of the present disclosure may particularly allow to provide for a low cost, low complexity and universally applicable sensor device, tool or system for in vitro testing, determination and/or quantification of one or more constituents of a test atmosphere, such as one or more inhalable substances, particles, molecules or other components of the test atmosphere. Therein, the sensor device according to the present disclosure may particularly address, at least mitigate or even overcome one or more of the drawbacks of conventional devices and systems utilized to measure or quantify exposure to a test atmosphere or its constituents, as described hereinabove.

For example, the sensor device described herein may particularly be applied or utilized to quantify exposure in an aerosol exposure application, for example to determine in vitro doses associated with the test atmosphere under investigation and/or one or more constituents thereof. This can comprise determination of administered dose, delivered dose and/or one or more measures correlating with one or more of the aforementioned dose quantities. It is emphasized, however, that utilization or application of the sensor device of the present disclosure is not limited to the determination of a dose or measure correlating therewith. In other words, the sensor device of the present disclosure is not limited to the actual determination of doses, dose values or a measure correlating therewith, but can be used to advantage in aerosol exposure systems to generally analyze or assess potential effects arising from or being associated with the exposure of the cell culture or the sensing device to the test atmosphere. Although not limited thereto, however, the sensor device according to the present disclosure may particularly allow for a simple, affordable, broadly applicable, sensitive and selective measurement and/or determination of the dose administered and/or delivered in an in-vitro exposure based on or using one or more receptor elements of one or more electrochemical sensors.

In the context of the present disclosure, the test atmosphere may generally refer to a test substance or medium comprising one or more gaseous, solid, and/or liquid constituents that may be detected, sensed, analyzed, recognized and/or investigated by means of the sensor device. The test atmosphere may be or comprise a gas, a mixture of gases, or a suspension of particles or aerosols in liquid and/or solid form in a gaseous matrix. Any gaseous, fluid, liquid and/or solid component of the test atmosphere, such as a gas molecule, an aerosol, a solid particle, or a liquid particle, may be referred to herein as constituent or component of the test atmosphere, which may be detected using the sensor device of the present disclosure. It is noted that the test atmosphere can contain an arbitrary number of constituents of one or more types, which can be sensed or detected by means of the sensor device of the present disclosure.

As used herein, the receptor element may generally refer to a sensing element of an electrochemical sensor, which receptor element may be configured to physically and/or chemically interact with one or more constituents of the test atmosphere to be sensed or investigated. For example, the receptor element may comprise a biologically derived material and/or biomimetic component configured to bind with, recognize and/or otherwise interact with the one or more constituents of the test atmosphere. In a non-limiting example, the receptor element may include at least one of a molecularly imprinted polymer, an antibody, a receptor protein, and an enzyme configured to interact with the one or more constituents in the test atmosphere.

The sensor device of the present disclosure can advantageously be used in conjunction with one or more receptor elements of one or more electrochemical sensors of one or more types. For instance, the one or more receptor elements may be at least partly inserted into the measurement compartment of the sensor device. Alternatively or additionally, one or more receptor elements of one or more electrochemical sensors may be pre-installed in the sensor device. Accordingly, the sensor device of the present disclosure may include one or more receptor elements and/or may be configured to receive one or more receptor elements of one or more electrochemical sensors, as will be further described hereinbelow.

The at least one transducer element of the sensor device can be functionally coupled to the at least one receptor element. For instance, the transducer element and the receptor element can be physically, chemically and/or mechanically coupled such that an interaction of the receptor element or a component thereof with one or more of the constituents of the test atmosphere results in a signal that can be detected by means of the transducer element and/or that can be converted by the transducer element into the electronically processible signal. Accordingly, the transducer element of the sensor device of the present disclosure may be configured to detect an interaction between the receptor element and the one or more constituents in the test atmosphere and convert the detected interaction into the electronically processable signal. Such detection of an interaction may include detection of an occurrence of the interaction, optionally per unit time. Accordingly, the electronically processible signal may be indicative of the occurrence or occurrence rate of interactions between the one or more receptor elements and the one or more constituents.

In an example, an interaction of the receptor element with one or more constituents of the test atmosphere can induce a configurational change, a conformational change, a compositional change, a positional change, a change of a chemical property, a change of a physical property and/or a change of another property or characteristic of the receptor element, which may be detectable with the transducer element. In a non-limiting example, an interaction between the receptor element and the one or more constituents may induce a modification and/or alteration of an electric field in the vicinity of the receptor element, which can be sensed and/or used by the transducer element to generate the electronically processible signal. Depending on the type of electrochemical sensor and/or receptor element used, also other detection principles, such as optical, piezoelectric, chemical, and/or chemiluminescence-based signals induced by interaction of the receptor element and the one or more constituents may be employed to detect the one or more constituents and generate the electronically processible signal.

As used herein, the electronically processible signal may refer to an electronic signal that can be processed by a processing circuitry, for example a processing circuitry of the sensor device or the aerosol exposure system. The electronically processible signal may be indicative of and/or contain information related to the presence of the one or more constituents in the test atmosphere, for example as detected using the transducer element based on detecting the interaction of the one or more constituents with the receptor element. Optionally, the electronically processible signal may be indicative of and/or contain information related to a dose associated with the one or more constituents, a delivered dose, an administered dose, a measure correlating with the dose, a measure correlating with the administered dose, a measure correlating with the delivered dose, a concentration of the constituents in the test atmosphere and/or other information associated with the exposure or allowing to quantify or characterize the exposure.

As used herein, the measurement compartment may refer to a at least partially closed volume, container or vessel of arbitrary shape and volume configured to at least partly encompass, receive and/or support the receptor element of the electrochemical sensor. The measurement compartment may have one or more open sides for inserting the receptor element into or removing it from the measurement compartment, for example at a side opposite to the side at which the receptor support is arranged. Optionally, the measurement compartment may be closable, for example by means of a cover or lid.

The receptor support may generally refer to a support structure or support, for example a solid support structure, configured to carry and/or hold the receptor element. In particular, the receptor support may be configured to position, fix, and/or arrange the receptor element, the transducer element and/or the electrochemical sensor within the measurement compartment, for example at a predefined position or height within the measurement compartment.

In the following, various aspects and associated advantages of the sensor device describe herein are exemplary summarized. The sensor device of the present disclosure may provide a structure which can allow for a reliable, simple and reproducible positioning of a receptor element or electrochemical sensor into the exposure chamber of an aerosol exposure system and exposing the sensor device to the test atmosphere comprising one or more test constituents, such as one or more aerosols, under conditions identical or vastly similar to the conditions under which cell cultures are exposed in an aerosol exposure system. Hence, a reading of the sensor device and/or the electronically processible signal may accurately reflect and/or quantify the exposure of the cell culture.

For this purpose, the sensor device comprises the transducer element, which may be functionally and/or operatively couplable to one or more receptor elements of one or more electrochemical sensors, for example based on contacting the receptor element arranged on the receptor support of the sensor device with the transducer element. The sensor device with the receptor support can be designed in such a way that a receptor element present on the receptor support is in functional contact with the transducer elements. Furthermore, the sensor device can be designed or configured in such a way that it can be placed into the exposure chamber of an in vitro aerosol exposure system.

In an example, the sensor device can be designed or configured in such a way that, for example when carrying a receptor element at the receptor support and when present in the exposure chamber of an in vitro aerosol exposure system, the position of the receptor element and/or a surface thereof with respect to a flow of the test atmosphere within the exposure chamber is equivalent and/or corresponds to the position of a cell culture surface during an exposure experiment within the same aerosol exposure system or chamber. Alternatively or additionally, the sensor device can be designed or configured in such a way that, when carrying a receptor element and when present in the exposure chamber of an in vitro aerosol exposure system, the test atmosphere flow conditions within the exposure chamber are identical or substantially correspond to the test atmosphere flow conditions in presence of a cell culture. For instance, the sensor device can be configured or designed in such a way that, when carrying a receptor and when inserted into an in vitro aerosol exposure system, the spatial geometry of the measurement compartment containing the receptor element substantially matches or is identical to the spatial geometry of the cavity a cell culture faces during in vitro aerosol exposures.

In an exemplary and non-limiting embodiment, the sensor device may correspond in size, shape and/or material to a standardized cell culture insert, for example a standardized 24-, 12- or 6 well transwell cell culture insert. Accordingly, the sensor device may be embodied as replica or reproduction of a 6-, 12-, or 24-well cell culture insert.

Optionally, the sensor device can be configured or designed in such a way that the transducer element and/or one or more receptor elements located in and/or on the sensor device can be connected to a power source, a processing circuitry, a processing device or other device or component of the sensor device or the aerosol exposure system, in particular without interfering with aerosol exposure system properties of functionality. Accordingly, the sensor device of the present disclosure may be compatible with existing in vitro aerosol exposure systems currently in use. The sensor device described herein can be simple to handle, can be reproducibly placed in the exposure chamber of an aerosol exposure system and can recapitulate the aerosol flow patterns above exposed cell cultures as well as sorptive and electrostatic properties of standard transwell inserts. Optionally, the receptor element may provide a surface that can be physically and/or chemically similar to the surface of a cell culture, such that the kinetics of aerosol deposition on the receptor element may be highly comparable to the kinetics of aerosol deposition on an exposed cell culture. This may allow for an accurate determination of the dose delivered or administered in the aerosol exposure.

The sensor device of the present disclosure can offer various advantages when compared to conventional systems utilizing OCMs for detection of constituents of the test atmosphere and quantification of the exposure of a cell culture to the test atmosphere, as described hereinabove. In particular, using electrochemical sensors and corresponding receptor elements can offer a much broader spectrum of potential target compounds or constituents that can be detected, and a high selectivity, in particular if molecularly imprinted polymers or antibodies are used as receptor elements. Furthermore, electrochemical sensors can be produced in very small sizes and in thicknesses far below one millimeter. Incorporation of electrochemical sensors or corresponding receptor elements into the exposure chambers can therefore be achieved for any in vitro aerosol exposure system without the need for re-designing the system or the sensor device. Hence, the senor device according to the present disclosure can be produced in larger numbers and at low costs, while allowing for immediate readout or readout within minutes and commonly do not require expensive instrumentation, which can render them highly time and cost efficient. Furthermore, the sensor device of the present disclosure is simple in operation and may not require highly qualified staff, for example when compared to post-hoc analyses utilizing highly sophisticated measurement equipment, such as electron microscopes.

In an example, the measurement compartment of the sensor device may define an interior volume, in particular a cylindrical interior volume, of the sensor device configured to least partly encompass the receptor element of the electrochemical sensor along a perimeter thereof. Accordingly, the measurement compartment may form an interior volume configured to at least partly, in particular entirely, contain and/or surround the receptor element along its perimeter. Optionally, also at least a part of the transducer element and/or at least a part of the receptor support may be contained or arranged in the measurement compartment. In such configuration of the measurement compartment, it may be ensured that the sensor device closely mimics the configuration of a cell culture in a standardized container for an aerosol exposure experiment, thereby allowing to accurately determine and optionally quantify the exposure.

In an example, the receptor support may define a bottom wall of the interior volume of the sensor device. In other words, the receptor support may be arranged at an end or side of the measurement compartment, which end or side constitutes a bottom wall of the measurement compartment. For example, the measurement compartment may comprise an elongated body, for instance a cylindrical body, wherein the receptor support may be arranged at a bottom end of the body of the measurement compartment.

Optionally, a top end of the measurement compartment opposite to the bottom end may be open to allow insertion into and removal of the receptor element or other components from the measurement compartment. Further optionally, the top end may be closable by means of a lid or cover for protecting the interior of the measurement compartment, for example for storing the sensor device. It is noted that orientations like "top" and "bottom" are intended to denote different parts or ends of the measurement compartment, in particular when the sensor device is oriented in an upright position in the Earth's gravity field as intended for use.

According to an example, the measurement compartment and the receptor support may together form a cavity of the sensor device configured to hold a matrix mimicking a surface of a cell culture, such as a cell culture medium, in particular a liquid and/or gelatinous cell culture medium, or another liquid, gelatinous, polymeric and/or porous material mimicking the surface of the cell culture. In other words, the receptor support and the measurement compartment may form a vessel or cavity defining an interior volume of the measurement compartment which may be configured to contain a matrix, for example a liquid or gelatinous matrix, mimicking or resembling the surface of a cell culture or cell culture medium thereof. Accordingly, the cavity formed by the measurement compartment and the receptor support may provide a substantially liquid or air-tight seal between the interior volume of the cavity and the surrounding of the sensor device. In such configuration, the surface of an actual cell culture may be closely resembled, mimicked and/or replicated by the measurement compartment, the receptor support and the matrix, such that a reading and/or the electronically processible signal of the sensor device allows to accurately determine and optionally quantify the exposure of the cell culture.

In an example, the receptor support and the measurement compartment may be integrally formed. In other words, the receptor support and the measurement compartment may be formed as a single part, for example from the same material. Such design may increase an overall robustness of the sensor device. Also, a manufacturing process may be simplified and production costs may be reduced.

In an alternative example, the receptor support may be fixedly attached to the end of the measurement compartment. For instance, the receptor support may be glued, welded, soldered or otherwise fixedly attached to the end of the measurement compartment, for example using a mechanical connection, such as a clamp, bolt or screw connection.

In an exemplary implementation, the measurement compartment and/or the sensor device is sized, shaped and formed in correspondence with an interior volume of an exposure chamber and/or a well of a culture plate, in particular a 6-, 12- or 24-well cell culture plate, such that at least a part of the measurement chamber is positionable within the exposure chamber and/or within the well of the cell culture plate. As discussed hereinabove, aerosol exposure experiments are typically performed using a 6-, 12-, or 24-well cell culture plate, where some or a subset of the wells contain cell cultures and some or a subset of wells contain sensors or are used to determine the exposure. By forming the measurement compartment to substantially match an interior volume of a multi-well cell culture plate, it may be ensured that the sensor device can be at least partly inserted into a well of the cell-culture plate, such that the measurement compartment and other components arranged therein, for example the receptor support, the receptor element and/or a gelatinous or liquid matrix, may accurately mimic the cell culture arranged in other wells of the cell culture plate, thereby ensuring accurate determination or quantification of the exposure of the cell cultures.

In an example, the measurement compartment and/or the sensor device may be sized, shaped and formed in correspondence with an interior volume of an exposure chamber and/or a well of a culture plate, in particular a 6-, 12- or 24-well cell culture plate, such that the receptor support and/or the receptor element of the electrochemical sensor is positionable at a predetermined position within the exposure chamber and/or within the well of the cell culture plate. The predetermined position within the exposure chamber and/or well of the cell culture plate may refer to a position, in which an outer surface of the sensor device within the measurement chamber, for example a surface of a gelatinous or liquid matrix covering the receptor element substantially corresponds to or is identical to a surface of a cell culture, cell culture overlay or cell culture medium covering a cell culture. Such positioning of the receptor support and hence the receptor element may ensure that the sensor device is exposed to the test atmosphere in a substantially identical manner as a cell culture that arranged in another well.

According to an example, the receptor support may be arranged and/or configured to position the receptor element relative to the transducer element, such that a functional contact between the transducer element and the receptor element of the electrochemical sensor can be established. As used herein, a functional contact may refer to or involve a relative arrangement of the transducer element and the receptor element allowing to sense an interaction between the receptor element and the one or more constituents of the test atmosphere. For instance, the receptor support may be configured to position the receptor element close to, in proximity of or in direct contact with the transducer element, such that a signal generated by the receptor element upon interaction with one or more constituents of the test atmosphere can be sensed or detected with the transducer element and converted into the electronically processible signal. For example, an interaction of the receptor element with one or more constituents may result in a chemically, electrically, electrostatically or otherwise detectable signal, and the receptor support may be configured to position the receptor element close enough to the transducer element to allow for a detection of such signal at the transducer element.

In an example, at least a part of the transducer element may be integrated into and/or embedded in the receptor support. Such configuration may increase mechanical robustness of the sensor device. Also, accuracy in the detection of the exposure may be enhanced, since accurate relative positioning of the transducer element, the receptor support, and optionally the receptor element can be ensured.

Alternatively or additionally, at least a part of the transducer element may be attached to a side of the receptor support facing an interior volume defined by the measurement compartment. For instance, at least a part of the transducer element may be arranged at a bottom side or wall of the measurement compartment. Optionally, at least a part of the transducer element may be arranged within the measurement compartment. For instance, at least a part of the transducer element may be arranged on an interior surface of the measurement compartment. This may allow to increase sensitivity of the sensor device, as the transducer element may gather signals generated close to its position within the measurement compartment.

In an exemplary implementation, the transducer element may include an array of electrodes arranged at a side of the receptor support facing the measurement compartment. Therein, the array of electrodes may include a working electrode, a counter electrode, and a reference electrode. However, the array may also comprise more than three electrodes. For instance, the array of electrodes may be arranged at an interior surface of a bottom wall or side of the measurement compartment, which interior surface may be directed towards the interior volume of the measurement compartment. The array of electrodes may be in direct contact with the interior volume of the measurement compartment or may be covered by a layer of material for protection.

Generally, the transducer element and/or the array of electrodes may be configured to detect and/or sense an interaction or occurrence of an interaction between a receptor element and one or more constituents of the test atmosphere based on detecting a change, modification or alteration of an electric field surrounding the array of electrodes. By way of example, the array of electrodes may be powered to generate an electric field around a part of the transducer element. When an interaction between a receptor element and a constituent occurs, for instance a conformational or other change may be induced in the receptor element, which may locally distort the electric field around the array of electrodes or change the ionic composition. The modification or distortion of the electric field may then be sensed by the transducer element and the electronically processible signal may be generated in response. Therein, the electrical signal detected by the transducer element in response to the interaction may constitute the electronically processible signal. Alternatively, the electronically processible signal may be generated based on the signal detected by the transducer element in response to the interaction, which may for example include amplification of the signal detected by the transducer element.

In yet a further example, the sensor device comprises at least one receptor element of an electrochemical sensor. Accordingly, the receptor element may be pre-installed in the sensor device, for example to provide a ready-to-use sensor device. Optionally, the sensor device may be activated before use, for example based on supplying a liquid and/or gelatinous matrix to the measurement compartment that mimics a cell culture.

As mentioned hereinabove, the receptor element of the electrochemical sensor may be configured to physically and/or chemically interact with the one or more constituents of the test atmosphere. For instance, an interaction of the receptor element with one or more constituents of the test atmosphere can induce a configurational change, a conformational change, a compositional change, a positional change, a change of a chemical property, a change of a physical property and/or a change of another property or characteristic of the receptor element, which may be detectable with the transducer element. Further, the transducer element may be configured to detect the interaction between the receptor element and the one or more constituents in the test atmosphere and convert the detected interaction into the electronically processable signal. This may optionally include processing and/or amplification of a signal detected by the transducer element in response to occurrence of the interaction between the receptor element and the one or more constituents.

In an example, the transducer element may be configured to transmit the electronically processable signal to a signal processing device. The transducer element may be communicatively and/or operatively coupled to the signal processing device (also referred to herein as processing device) to transmit the electronically processible to the signal processing device. Therein, the coupling may be a wired or wireless coupling.

The signal processing device may be part of the sensor device. Alternatively or additionally, the signal processing device may be part of an aerosol exposure system. Generally, the signal processing device may comprise a processing circuitry with one or more processors for data processing. The processing circuitry may also include other components, such as an amplification circuitry to amplify a signal received at the transducer element in response to the interaction between the receptor element and the one or more constituents.

In a non-limiting example, the measurement compartment and/or the receptor support may comprise one or more of glass, polystyrene, polyethylene terephthalate, polydimethylsiloxane, stainless steel, Polyetheretherketon, Teflon, and Silicon. Also other materials or material compositions can be used. Such materials may be particularly advantageous as they are non-reactive or inert and may not adversely affect or influence the actual measurement.

In yet another example, the receptor element may include at least one of a molecular imprinted polymer, an antibody, a receptor protein, and an enzyme configured to interact with the one or more constituents in the test atmosphere. For instance, the receptor element may include a layer of at least one of a molecular imprinted polymer, an antibody, a receptor protein, and an enzyme configured to interact with the one or more constituents in the test atmosphere. Such receptor elements may provide high selectivity and high sensitivity for detecting the one or more constituents in the test atmosphere. Hence, the aerosol exposure can be reliably and accurately determined, and optionally also quantified, for example based on determining a dose, an administered dose, a measure correlating with a dose, and/or a delivered dose.

Moreover, such receptor elements can be produced in very small sizes and in thicknesses far below one millimeter. Incorporation of electrochemical sensors or corresponding receptor elements into the exposure chambers can therefore be achieved for any in vitro aerosol exposure systems without the need for re-designing the system or the sensors.

In yet another example, the sensor device may further comprise a layer of liquid and/or gelatinous matrix at least partly covering the molecular imprinted polymer, the antibody, the receptor protein, and/or the enzyme. Optionally, the layer of liquid and/or gelatinous matrix may provide a physical and chemical environment that may be suitable for the functioning of the receptor element and/or the transducer element. For instance, if a conductive bridge between working and counter electrodes should be established, the matrix layer may be conductive and may be adapted with respect to its electrical properties. Alternatively or additionally, the layer of liquid and/or gelatinous matrix may provide a physical and chemical environment that resembles the surface of a cell culture cultivated at the ALI. For instance, the matrix layer may be adapted with respect to a polarity of a solvent, the surface tension, and/or the pH value.

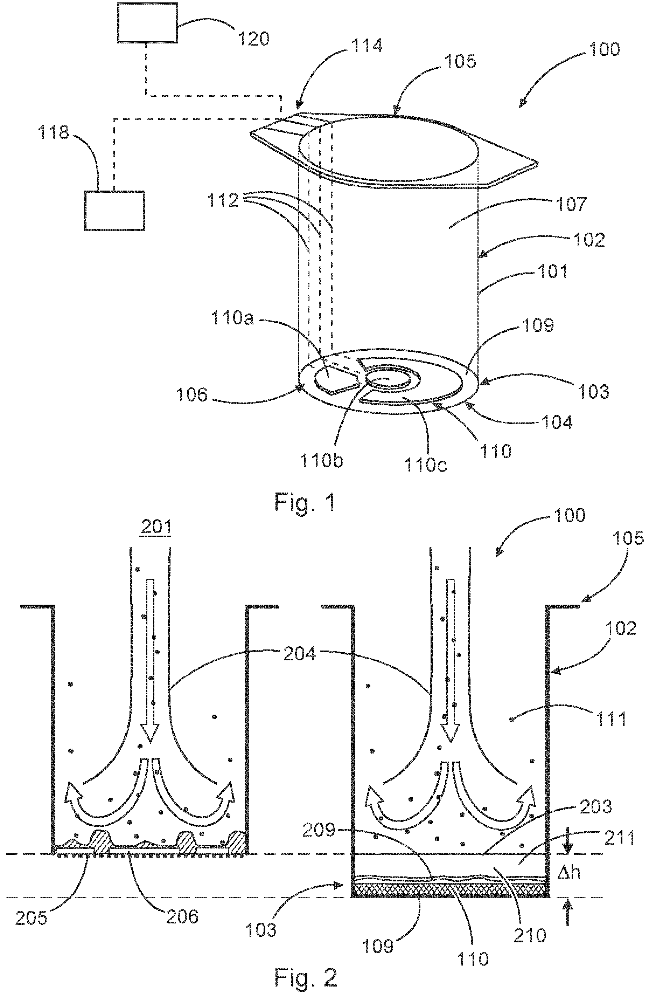

Optionally, the matrix layer may be considered a part of the sensor devices receptor element and a dimension of the sensor device may take into account the thickness of this liquid layer, so that the location of the surface of the receptor element may be aligned with a location of the cell culture surface with respect to the geometry of the exposure chamber, as exemplary described in detail with reference to Figure 2.

Generally, a type and number of receptor elements may be chosen in dependence of the actual application or the one or more constituents under investigation. Since one or more receptor elements of same or different type can be chosen, the sensor device described herein may constitute a modular sensor system for in-vitro aerosol exposure systems. Also, at least a part of the sensor device may be re-used multiple times, for example with different receptor elements.

In an exemplary embodiment, the receptor element may include a plurality of molecular imprinted polymers, antibodies, receptor proteins and/or enzymes of different types, each type being configured to interact with a different type of constituent in the test atmosphere. Using different types of receptor elements for detecting different types of constituents may allow to detect a broad spectrum of constituents with high selectivity and sensitivity.

In an example, the transducer element and/or the receptor support may be configured to directly contact at least a part of the receptor element. For example, the receptor element and/or a material thereof may be deposited directly onto at least a part of the receptor support, for instance based on pipetting receptor material into the measurement compartment or another deposition technique. By contacting the receptor element and the transducer element, it may be ensured that an interaction between the receptor element and the one or more constituents can be detected with high accuracy and precision. Accordingly, a sensitivity of the sensor device may be increased.

In yet a further exemplary implementation, the sensor device may further comprise an interface for coupling the sensor device to a power supply and/or for coupling the sensor device to a processing device or signal processing device. Therein, the power supply and/or processing device may be part of the sensor device or may be part of an aerosol exposure system. The interface may be configured for electrically coupling the sensor device to the power supply and/or for communicatively coupling the sensor device to the processing device. One or both the electronic and the communicative coupling may be a wired or wireless. Wireless electronic coupling may, for example, involve inductive and/or capacitive coupling.

In an example, the interface may be configured for independent readout of signals of a plurality of receptor elements and/or transducer elements. Accordingly, the interface may be communicatively coupled to a plurality of transducer elements and/or receptor elements of same or different type and independently acquire a plurality of measurement signals, each signal corresponding to one or a subset of receptor/transducer elements of the plurality of receptor/transducer elements. The plurality of measurement signals may then form the electronically processible signal. Alternatively, each of the independent measurement signals may constitute an electronically processible signal. In particular, an independent readout can improve selectivity of the sensor device. Also, a spectrum of detectable constituents may be increased.

According to an example, the interface may be configured for one or more of inductive coupling, electronic coupling, capacitive coupling, and optical coupling. Any one or more of the aforementioned couplings may be used to couple the sensor device to the power supply and/or to the processing device.

In yet another example, the sensor device further comprises one or more electrical leads or wires contacting the transducer element for power supply and/or for transmission of the electronically processible signal to a processing device. Using electrical leads or wires for power supply and communication or transmission of the electronically possible signal may allow to establish a mechanically robust connection.

In an exemplary implementation, the one or more electrical leads or wires may extend from the transducer element along a wall, in particular an outer wall, of the measurement compartment towards a further end of the measurement compartment. The further end of the measurement compartment may denote a top end of the measurement compartment arranged opposite to a bottom end of the measurement compartment, where the receptor support may be located. The electrical leads or wires may be arranged on a surface of the measurement compartment, for example on an outer surface of a body of the measurement compartment that is directed away from the interior volume of the measurement compartment. Optionally, the leads may be covered by insulating material for protection. Alternatively or additionally, at least a part of the electrical leads may extend through a wall of the measurement compartment. For instance, the electrical leads may be at least partly embedded or integrated in the outer wall of the measurement compartment or a body thereof.

In yet another example, the sensor device further comprises a holder configured to receive at least a part of the measurement compartment. In particular, the measurement compartment may be placed into the holder after exposure to the test atmosphere, for example for post-hoc readout.

Optionally, the holder may include an interface configured to establish an electronic connection with the transducer element upon receipt of the measurement compartment in the holder. In particular, an electronic coupling between the transducer element and the power source may be established. Alternatively or additionally, a communicative coupling between the transducer element and a processing device may be established.

Further optionally, the holder may provide one or more guides, for example on a surface thereof, which may be formed in correspondence with one guides arranged on a surface of the measurement compartment, such that the measurement compartment is insertable into the holder in a predefined orientation, in which the leads of the measurement compartment may be aligned with the interface of the holder. Generally, such configuration and design may simplify handling and operation of the sensor device.

In a non-limiting example, the transducer element may comprise one or more of gold, platinum and glassy carbon. In particular one or more electrodes of the transducer element may comprise one or more of the aforementioned materials, as these may be considered highly conductive, but chemically non-reactive materials. Also other materials can be used, which should preferably not alter or affect a physical and chemical environment of the transducer element or a receptor element arranged in its vicinity.

According to an example, the transducer element may be configured for one or more of voltametric, amperometric and impediometric readout. Alternatively or additionally, the electronically processible signal may be read from the transducer element, for example by a processing device, based on voltametric, amperometric and/or impediometric readout.

In an example, the sensor device further comprises a processing device configured to process the electronically processible signal provided by the transducer element into a measure correlating with a dose associated with the one or more constituents of the test atmosphere. Therein, the measure correlating with the dose may refer to a physical quantity indicative of and/or proportional to the dose associated with the exposure and/or the one or more constituents. Accordingly, the processing device may be configured to quantify the exposure based on processing the electronically processible signal and computing the dose associated with the exposure or the one or more constituents.

The processing device may be configured to determine and/or compute an administered dose indicative of a mass of the one or more constituents of the test atmosphere supplied to an exposure chamber of an aerosol exposure system. The processing device may optionally take one or more parameters related to the exposure and/or the one or more constituents into account for computing the administered dose.

Alternatively or additionally, the processing device may be configured to determine and/or compute a delivered dose indicative of a mass of the one or more constituents of the test atmosphere deposited onto and/or transferred into the receptor element. As discussed hereinabove, the administered dose may allow to derive reliable information related to a response, for example a biological or biochemical response, of a cell culture to the one or more constituents of the test atmosphere

Optionally, the processing device may be configured to determine the measure correlating with the dose, for example correlating with the administered and/or delivered dose, based on one or more of a particle size of the one or more constituents, a deposition kinetics of the one or more constituents, a molecular mass of the one or more constituents, a volatility of the one or more constituents, a solubility of the one or more constituents, a volume flow rate through the exposure chamber, an exposure duration, and a composition of the test atmosphere. Taking one or more of the aforementioned parameters related to the exposure and/or the one or more constituents into account may allow to compute the measure correlating with the dose with accuracy and precision.

Further optionally, the processing device may be configured to determine a biochemical and/or biological response of the receptor element to the one or more constituents. Such biochemical and/or biological response may be determined based on the determined measure correlating with the dose, in particular based on an administered dose. Determining the biological and/or biochemical response may allow to assess safety of the one or more constituents for the respective cell culture or the cells thereof, and hence may allow to assess safety for humans, for example if the one or more constituents under investigation are inhalable substances.

In an example, the processing device may be configured for on-line monitoring and/or for post-hoc analysis of an in-vitro exposure of a cell culture to the test atmosphere. For instance, the processing device may read or receive one or more electronically processible signals from the transducer element during the exposure, thereby allowing for online monitoring of the exposure in real-time or near real-time. Alternatively or additionally, the processing device may read-out the transducer element after the exposure and the corresponding electronically processible signal may be indicative of all interactions between the receptor element and one or more constituents that occurred during the exposure.

A further aspect of the present disclosure relates to an aerosol exposure system for an in-vitro exposure of a cell culture to a test atmosphere. Any disclosure presented hereinabove and hereinbelow with reference to the sensor device equally applies to the aerosol exposure system, and vice versa. The system comprises one or more sensor devices, as described hereinabove and hereinbelow. The system further comprises a processing device or signal processing device for processing the electronically processable signal generated by the one or more sensor devices. Therein, the processing device may be operatively and/or communicatively coupled to the at least one sensor device.

In an example, the exposure system may include a 6-, 12-, or 24- cell culture plate, wherein some or a subset of the wells may contain cell cultures and one or more wells may contain one or more sensor devices, as described hereinabove and hereinbelow. The cell culture plate may be placed into an exposure chamber of the exposure systems and may be exposed to the test atmosphere. Since the one or more sensor devices mimic or resemble the cell cultures in terms of position, exposure geometry, physical properties, and/or chemical properties, the electronically processible signals gathered from the one or more sensor devices allow to accurately determine or compute the exposure of the cell cultures, which may optionally include quantification of the exposure, for example based on determining a measure correlating with the dose.

In an example, the system further includes an exposure trumpet for guiding a flow of the test atmosphere into the measurement compartment of the one or more sensor devices. By means of the trumpet the flow of test atmosphere may be controlled in terms of flow rate, volume rate and/or spatial exposure, thereby allowing to perform the exposure under controlled and/or reproducible conditions.

In an example implementation, the processing device may be configured for independent readout of a plurality of sensor devices of the system. For instance, a plurality of sensor devices of the same or different type, for example employing different types of receptor elements, may be utilized. Using a plurality of sensor devices that can be read out independently may particularly allow to detect a broad spectrum of different constituents, for example when using different types of sensor devices with different types of receptor elements.

A further aspect of the present disclosure relates to a use of a sensor device, as described hereinabove and hereinbelow, and/or an aerosol exposure system, as described hereinabove and hereinbelow, for determining and/or quantifying the exposure of a cell culture to a test atmosphere, in particular for dose assessment.

According to a further aspect of the present disclosure, there is provided a method of determining in-vitro exposure of a surrogate for a cell culture, such as for example a liquid and/or gelatinous cell culture medium, to a test atmosphere with one or more constituents under investigation. The method comprises:
- providing a sensor device as described hereinabove and hereinbelow;
- placing a receptor element of an electrochemical sensor into the measurement compartment of the sensor device;
- exposing the sensor device and the receptor element to the test atmosphere; and
- generating, with a transducer element of the sensor device, an electronically processable signal indicative of the one or more constituents of the test atmosphere.

The method may optionally comprise determining, based on processing the generated signal with a processing device of the sensor device or of the aerosol exposure system, a dose associated with the one or more constituents of the test atmosphere and/or a measure correlating with the dose.

Examples will now be further described with reference to the Figures in which:
Figure 1 shows a perspective view of a sensor device for an aerosol exposure system;
Figure 2 shows a cell culture insert in comparison with a sensor device for an aerosol exposure system;
Figure 3 shows an aerosol exposure system; and
Figure 4 shows a flow chart illustrating a method of determining in-vitro exposure of a surrogate for a cell culture.

The Figures are schematic only and not true to scale. In principle, identical or like parts, elements and/or steps are provided with identical or like reference numerals in the figures.

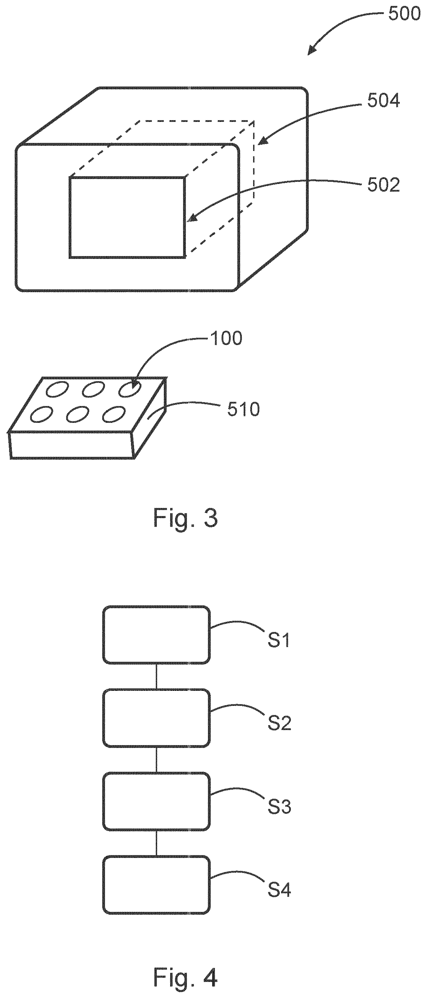

Figure 1 shows a perspective view of a sensor device 100 for an aerosol exposure system 500 (see Figure 3) according to an example.

The sensor device 100 comprises a measurement compartment 102 for at least partly receiving, encompassing and/or enclosing a receptor element (209, see Figure 2) of a biochemical sensor that can be placed into the measurement compartment 102 for determining the exposure. The measurement compartment 102 comprises an open end 105 or top end 105 via which the receptor element 209 and optionally other components can be inserted into or removed from the measurement compartment 102. The measurement compartment 102 further comprises a substantially cylindrical body 101 forming or defining an interior volume 107, into which the receptor element 209 can be placed for measurement of the exposure to the test atmosphere.

At a bottom end 103 opposite to the top end 105 with respect to a longitudinal axis of the measurement compartment 102 or sensor device 100, a receptor support 104 is arranged which is configured to support, hold and/or position the receptor element 209 within the interior volume 107 of the measurement compartment 102. The receptor support 104 may define a bottom wall 109 or bottom side 109 of the measurement compartment 102.

In the example illustrated in Figure 1, the receptor support 104 is integrally formed with the body 101 of the measurement compartment 102. Alternatively, the receptor support 104 may be fixedly attached to the body 101 by an appropriate connection, such as a clamp connection, screw connection, glue connection, welding or soldering.

The sensor device 100 further comprises a transducer element 106. In the example of Figure 1, the transducer element 106 comprises an array 110 of three electrodes 110a, 110b, 110c, and specifically a counter electrode 110b, a reference electrode 110a and a working electrode 110c. In the example shown in Figure 1, the electrodes may be plane or flat electrodes, wherein the counter electrode 110b may be arranged in a center of the array 110 and the working electrode 110c and the reference electrode 110a may be arranged around a part of a perimeter of the counter electrode 110b to provide a highly sensitive electrode array 110.

The transducer element 106 of the example of Figure 1 is arranged at the bottom wall 109 of the measurement compartment 102 defined or formed by the receptor support 104. For instance, the transducer element 106 may be arranged on an interior surface of the bottom wall 109 facing the interior volume 107 of the measurement compartment 102. The transducer element 106 and/or the array 110 of electrodes 110a-c may for example be printed or glued onto the interior surface of the bottom wall 109. Optionally, the electrode array 110 may be covered by a layer of protective material. The transducer element 106 and/or the electrode array 110 may comprise one or more of gold, platinum, and glassy carbon.

The sensor device 102 further comprises a plurality of electrical leads 112, wherein each of the electrodes 110a-c is coupled to at least one of the leads 112. The leads 112 extend from the array of electrodes 110 and/or the bottom wall 109 towards the top end 105 or a rim located at the top end 105. Therein, the leads 112 are configured to electrically couple the sensor device 100 to a power supply 118 and/or to communicatively couple the sensor device 100 or transducer element 106 to a processing device 120 configured to process one or more electronically processible signals generated by the transducer element 106 in response to the interaction of the receptor element 209 with one or more constituents in the test atmosphere, as described in more detail hereinabove and hereinbelow. The power supply 118 and/or the processing device 120 may be part of the sensor device 100 or may be part of an aerosol exposure system 500, to which the sensor device 100 can be connected.

To actually couple the sensor device 100 to the power supply 118 and the processing device 120, the leads 112 of the sensor device 100 are coupled to or form an interface 114 of the sensor device 100 arranged at the top end 105 of the sensor device 100.

In the following, various aspects of the sensor device 100 are described and summarized. The sensor device 100 and/or the measurement compartment 102 may refer to a solid structure which in size, shape and material may be replicate or mimic standardized 24-, 12- or 6- well cell culture inserts. The electrodes array 110 may be located on the inner side or surface of the bottom wall 109 of the measurement compartment 102, which may be referred to as transwell replica. The array 110 includes a working electrode 110c, a counter electrode 110b, and a reference electrode 110a. The electrode array 110 may be part of the transducer element 106 couplable to a receptor element 209 of an electrochemical sensor. The receptor element 209 of an electrochemical sensor, for instance a molecularly imprinted polymer or other receptor element, can be deposited directly onto the electrodes 110a-c. Electrical leads 112 may be present in or on a side wall of the measurement compartment 102 or the body 101 thereof. The electrical leads 112 may lead to the top end 105 or rim of the measurement compartment 102 or sensor device 100. An interface 114 or means for establishing electrical connections to a power supply 118 and/or processing device 120, such as a Potentiostat, Galvanostat, and/or Impedance Analyzer, is provided at the top end 105 of the sensor device 100. Optionally a receptor element 209 of an electrochemical sensor may be arranged in the measurement compartment 102.

The receptor element 209 may preferably be in direct contact with the electrode array 110 and/or the transducer element 106. Optionally, the receptor element 209 may comprise a thin functional layer, such as a layer of a molecularly imprinted polymer, immobilized antibodies, immobilized enzymes, proteins, or other material as described herein.

Further optionally, the sensor device 100 may comprise a holder (not shown) for post-hoc readout. For instance, the holder may be configured to at least partly receive the measurement compartment 102 after exposure. The holder may comprise an electrical connector or interface, which, upon insertion of the measurement compartment 102 may establish a connection between the transducer element 106, the interface 114 and/or the electrical leads 112, and optionally a power source 118 and/or processing device 120. The holder may be shaped in correspondence with the measurement compartment 102, such that the measurement compartment 102 may tightly fit into the holder. Optionally, the holder may provide one or more guides configured to engage with one or more correspondingly shaped guiding elements or guides arranged at the measurement compartment 102 or an outer surface thereof, so that the orientation of the measurement compartment 102 may for example by default be aligned with the electrical connections or interface present on the holder.

The measurement compartment 102 can be made from any suitable material, such as for instance glass, polystyrene, polyethylene terephthalate, and polydimethylsiloxane. Depending on the chosen material, it can be produced by machining, additive manufacturing or injection molding, wherein optionally at least a part of the transducer element 106 may be embedded in the measurement compartment 102 or a wall thereof.

The electrodes 110a-c and the electrical leads 112 can be made from any suitable material that is compatible with electrochemical applications, such as for example gold, platinum, and glassy carbon, and may be mounted in any feasible way, such as by gluing of individual electrodes 110a-c or leads 112 or the complete electrode-lead array 110, 112 on the bottom wall 109 of the measurement compartment 102, for example by screen printing.

The receptor element 209 may be deposited in the measurement compartment 102, for example onto the electrodes 110a-c by the user so that selectivity of the sensor device 100 can be custom tailored in a modular fashion. In such instances, the sensor device 100 may comprise the measurement compartment 102, the transducer element 106, the receptor support 104 and optionally the electrical leads 112 and/or the interface 114.

In an alternative configuration, the sensor device 100 may be ready-to-use, wherein the receptor element 209 may be present and in functional connection with the transducer element 106. In such cases, the target analyte or constituent of the test atmosphere to be detected by the sensor device 100 may be predefined.

The receptor element 209 can be deposited on the receptor support 104 by any suitable method, including manual deposition e.g. by pipetting, and may optionally be followed by thermal or UV curing. Other deposition techniques may include spraying, printing, spin-coating, and electro polymerization.

The receptor element 209 may, for instance be a bioreceptor, such as a receptor based on nucleic acids, antibodies, proteins or enzymes, or a molecularly imprinted polymer (MIP). The readout may be, for instance, voltametric, amperometric or impediometric. Specific sensor parameters, such as for example sensitivity, selectivity, and dynamic range, may depend on the analyte or constituent of interest, i.e. the targeted constituent of the test atmosphere under investigation.

One or more types of receptor elements 209 may be present in the same measurement compartment 102, for example if several test atmosphere constituents are to be targeted at the same time in a compartment 102. Separate electrical connections 112, 114 may be provided for each receptor element 209, analogously to the connections provided for a single receptor element 209.

In use, one or more measurement compartments 102 can be placed into one or more exposure chambers of an aerosol exposure system 500 (see Figure 3), and the system 500 may be closed and operated as during normal aerosol exposure experiments. If the system 500 provides several exposure chambers, chambers not occupied by sensors may be used for cell culture exposures, as the presence of the sensors in the other chambers does not interfere with system functions.

Further, the sensor device may be operated in two different modes of operation. Depending on the exposure system 500, electrical connections to the sensor device 100 may be present in the exposure system 500 during the exposure, offering the possibility of performing real-time or on-line measurements of test atmosphere deposition. Alternatively, for example if the exposure system 500 does not allow for including electrical connections into the exposure chambers, post-hoc readout may be performed. After completing the exposure, the sensor device 100 or measurement compartment 102 containing the receptor elements 209 and the test atmosphere constituents that deposited onto and/or into it during the exposure can be removed from the system 500, connected to the power supply 118 and processing device 120 and the mass deposition of the targeted test atmosphere constituents may be measured.

The holders onto which the measurement compartments 102 may optionally be placed for this purpose may be located in a sealable containment within which the physical and chemical conditions can be controlled in order to provide optimal conditions for the readout and to avoid the generation of artifacts and contaminations. For instance, a protective atmosphere may be provided, or the temperature may be kept in the range optimal for the sensor.

Summarizing, the sensor device 100 described herein may provide a physical receptor support 104, which can bring the receptor element 209 into a position within an aerosol exposure system 500 that is equivalent to the position of cell cultures during in vitro exposures, for instance to inhalable test substances, as well as preferably the means or interface 114 for connecting the sensor device 100 to a processing device 120, which may convert the sensor output or electronically processible signal into a human readable and interpretable result. The receptor support 104 may comprise the transducer element 104 or parts of the transducer element 106. Optionally, the sensor device 100 may include a receptor element 209 arranged on the support 104 and/or the transducer element 106, as described in more detail with reference to Figure 2.

Figure 2 shows a standard cell culture insert 201 on the left-hand side of the figure in comparison with a sensor device 100 for an aerosol exposure system as proposed herein shown on the right-hand side of Figure 2. Unless stated otherwise, the sensor device 100 of Figure 2 comprises the same elements, features and functionalities as the sensor device 100 of Figure 1.

In the exemplary implementation of the sensor device 100 shown in Figure 2, a solid receptor support 104 may be arranged at the bottom end 103 of the measurement compartment 102, onto which the receptor element 209 of an electrochemical sensor can be deposited. The receptor support 104 may form the bottom wall 109 of the measurement compartment 102, where in the standard transwell cell culture insert 201, a porous membrane 205 is located. The format of the sensor device 100 may correspond to the 6, 12 or 24 well format of a standard transwell cell culture insert 201.

Depending on the surface properties and the functional principle of the receptor element 209, a thin layer 210 of a suitable liquid and/or gelatinous matrix 211 may be deposited on top of the receptor element 209. Preferably this layer 210 may provide the physical and chemical environment that may be suitable for the functioning of the sensor device 100, for instance for providing a conductive bridge between the working and counter electrodes 110a-c. Further, the layer 210 may resemble the surface of a cell culture cultivated at the ALI, for instance with respect to the polarity of the solvent, the surface tension, and/or the pH. In this case, the layer 210 may be considered a part of the receptor element 209 and the dimension of the measurement compartment 102 may take into account the thickness Δh of this liquid layer 210, the thickness of the receptor element 209, the thickness of the receptor support 104 and the thickness of the electrode array 110 or transducer element 106, so that the location of the receptor element's 209 surface 203 may be aligned with the location of the cell culture surface 206 with respect to the geometry of the exposure chamber. Accordingly, a height of the sensor device 100 measured from the bottom end 103 to the top end 105 may be larger than the height of the standard cell culture insert 201 by the sum of the thicknesses of the matrix layer 210, the receptor element 209, the electrode array 110, and the receptor support 104. In other words, the height of the standard transwell cell culture insert 201 and the height of the sensor device 100 or its measurement compartment 102 may differ by Δh, which may be the sum of the thickness of the receptor support 104, the transducer element 1906, the receptor element 209 and the liquid or gelatinous matrix layer 210 potentially present on the receptor element 209.

Generally, the sensor device 100 may display physicochemical properties similar to the ones displayed by the actual cell culture inserts 201, for example in terms of sorptivity and/or electrostatic properties. The measurement compartment 102 may therefore preferably be made from the same material as the standard transwell cell culture insert, which typically includes polystyrene.

As shown in Figure 2, the sensor device 100 may provide a sensing surface 203, which is substantially aligned with a sensing surface of a cell culture in the standard cell culture insert 201. The two inserts are shown in Figure 2 as if located in a typical in vitro aerosol exposure system 500, in which an exposure trumpet 204 projects into the cell culture inserts 210 or measurement compartment 102 and brings a test atmosphere with one or more constituents 111 under investigation into close proximity of the cell culture surface and/or sensing surface 203. In Figure 2, the airflow pathway is indicated by the arrows, and the test atmosphere or its constituents by the dots 111. The exposure chamber housing, which may surround the insert 201 and the sensor device 100 and the cell culture medium, which may be located below the insert 201, are omitted in the Figure 2 (see Figure 3).

As mentioned above, the bottom of the standard cell culture insert 201 is a porous membrane 205 on which a cell culture 206 is cultivated. The bottom wall 109 of the sensor device 100, on the other hand, provides a closed surface 109 on which an electrode array 110 and/or the transducer element 106 is arranged.

As discussed above, a height of the standard cell culture insert 201 and a height of the sensor device 100 or its measurement compartment 102 may differ by Δh, which may be the sum of the thickness of the receptor support 104, the transducer element 106, the receptor element 209 and the liquid or gelatinous matrix layer 210 potentially present on the receptor element 209. The vertical location of the surface 203 at which exposure takes place and the corresponding surface of the cell culture 206 in the standard cell culture insert 201 may be identical in the two configurations, wherein the thickness of the cell culture 206 and the porous PET membrane 205 in the standard transwell cell culture insert 201 may be negligible. Further, a distance between the exposed surface 203 (or corresponding surface of the cell culture 206) and the exposure trumpet 204 may therefore be identical in both configurations. Optionally all other aspects of the geometry of the cell culture inserts 201 may be similar or identical to the measurement compartment 102, such that the test atmosphere flow patterns and deposition kinetics may be the same or similar and the dose delivery to cell cultures and the sensor device 100 may also be identical or at least highly similar. Hence, the reading of the sensor device 100 may allow to accurately determine the dose associated with the exposure of a cell culture by the test atmosphere.

Depending on the type of constituent 111 under investigation and depending on the spectrum or range of constituents to be covered, also the sensor device 100 may be adapted, for example in terms of receptor elements 209 or types of receptor elements 209 chosen. Further, the sensor device 100 may be adapted depending on the needed selectivity and sensitivity and the physical and chemical environment in which it will be operated. Accordingly, the sensor device 100 described herein may provide for a highly adaptive and modular sensor system.

Preferred embodiments or examples may provide an electrochemical sensor, the receptor element 209 of which may be a molecularly imprinted polymer ("MIP"). The receptor support 104 onto which the MIP may be deposited, i.e. the bottom wall 109 of the measurement compartment 102 may comprise the electrode array 110 providing the working electrode 110c, the counter 110b electrode and the reference electrode 110a connected to a power source 118 and a processing device 120, such as a voltmeter, ampermeter, or impediometer and a processing circuitry with one or more processors for data processing. The MIP may preferably be deposited directly onto the electrodes 110a-c. Electrical connections to the power source 118 and/or processing device 120 may be present in the wall of the measurement compartment 102. The electrodes 110a-c and the electrical connections 114 can be, for instance, glued or printed into/onto the measurement compartment 102.

For reasons of sustainability, the sensor device 100 may be re-usable. MIPs can be regenerated several times, i.e. the bound target molecules can be removed from the MIP, for instance by washing in adequate solvents. In addition, if the specificity and/or selectivity of the MIP has decreased after several cycles of regeneration, the MIP can be removed from the surface of the receptor support 104 and new MIP can be deposited onto it. Polystyrene may be resistant to most common chemical and physical procedures used for MIP deposition, regeneration and removal, but measurement compartments 102 of other, more resistant materials, such as stainless steel, Teflon, PEEK, or coated measurement compartments 102, for example with gold, platinum, or Teflon, may be used for special applications in order to maintain reusability.

In the following, possible materials and dimensions for one or more components of the sensor device 100 are exemplary summarized. The measurement compartment 102 may include any material that can be brought into the desired shape and is of at least the stability of the actual cell culture vessel of a standard cell culture insert 201. Preferably the same material as the commercially available transwell inserts 201, such as polystyrene, may be used, as this may allow simulating any unforeseen material effects, for instance related to sorptivity. Porous materials or materials with large surface areas, such as rough structures, should be avoided, as this may increase losses of test atmosphere constituents. Further, for re-usable sensor devices 100, stainless steel or other materials such as PEEK that are not sensitive to solvents or heat (cleaning/autoclaving) be may be considered

For the optional receptor element 209, preferably a receptor element 209 compatible with electrochemical detection, such as molecularly imprinted polymers, antibodies, a receptor protein or enzymes may be used.

For the electrodes 110a-c, the transducer element 106, the electrical leads 112 and/or the interface 114, gold, platinum, or glassy carbon may be chosen. To maintain a broad application range and the possibility to regenerate and replace receptor elements 209, the electrodes 110a-c may be inert to mild acids and bases, biological fluids, and mild organic solvents.

Internal dimensions of the measurement compartment 102 may correspond in shape and dimension to the commercially available transwell cell culture inserts 201 of the standard 24, 12 or 6 well formats. A certain additional volume, specifically a larger depth or height, may be provided for the sensor devices 100 when compared to the standard inserts 201, in particular if the sensor device 100 is configured for a liquid environment, as is the case with electrochemical sensors and corresponding receptor elements 209. In this case, the measurement compartment 102 and/or sensor device 100 may be slightly deeper than the actual cell culture vessel of a standard insert 201 in order to bring the surface of the liquid or gelatinous matrix 210 to a position equivalent or corresponding to the cell culture surface (see Figure 2). Also the external dimensions of the sensor device 100 and/or measurement compartment 102 may preferably be similar to the dimensions of standard inserts 201, which may ensure compatibility of the sensor device 100 with the large majority of available exposure systems 500.

The electrical leads 112 and electrodes 110a-c may be adapted in size corresponding to a size of the measurement compartment 102.

In embodiments with electrical contacts 114 or the interface 114, for example where no wires may be attached to the top rim 105 or interface 114 of the measurement compartment 102, a connection to a power source 118 and/or processing device 120 may be established by plugging wires or the holder for post-hoc readout directly at the top rim of the measurement compartment 102. Preferably, the contacts should not interfere with insertion of the measurement compartment 102 into exposure systems 500 or cell culture plates and should therefore project from the measurement compartment 102 as little as possible. In embodiments with wires attached to the top rim of the sensor device 100 or measurement compartment 102, the wires should preferably be about 25cm in length to bridge the gasket sealing the exposure chambers of commonly used aerosol exposure systems 500 from the environment and to allow easy handling of the connection outside the exposure systems 500. The wires should be as thin as possible to not interfere with system tightness, and can preferably be designed as foil cables.

Figure 3 shows an aerosol exposure system 500 with one or more sensor devices 100, for example one or more sensor devices 100 as described with reference to Figures 1 and 2.

The aerosol exposure system 500 comprises an opening 502 via which the sensor device 100 may be inserted into an exposure chamber 504 of the system 500.

In the example shown in Figure 3, one or more sensor device 100 may be arranged in one or more wells of a multi-well or 6-well cell culture plate 510. Other configurations of culture plates, such as 12- or 24-well plates, may be used in the alternative.

The exposure system 500 may further comprise one or more exposure trumpets 204 as illustrated in Figure 2.

Further, the exposure system 500 may include a power source 118 or supply 118 and/or a processing device 120 for data processing, which may be communicatively and operatively coupled to the one or more sensor devices 100.

Figure 4 shows a flow chart illustrating a method of determining in-vitro exposure of a surrogate for a cell culture, such as a liquid and/or gelatinous cell culture medium, to a test atmosphere with one or more constituents 111 under investigation.

At step S1, a sensor device 100 is provided, for example a sensor device 100 as described with reference to Figures 1 and 2.

Step S2 comprises placing a receptor element 209 of an electrochemical sensor into the measurement compartment 102 of the sensor device 100. Optionally, the sensor device may be placed into an exposure chamber 504 of an exposure system 500.

Step S3 comprises exposing the sensor device 100 and the receptor element 209 to the test atmosphere, for example in an exposure chamber 504 of an exposure system 500.

Further, step S4 comprises generating, with a transducer element 106 of the sensor device 100, an electronically processable signal indicative of the one or more constituents 111 of the test atmosphere.

Optionally, the generated signal may be processed with a processing device 120 of the sensor device 100 or of the aerosol exposure system 500 and the exposure may be quantified, for example based on determining a measure correlating with a dose associated with the one or more constituents of the test atmosphere.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A sensor device (100) for an aerosol exposure system for in-vitro exposure of a cell culture to a test atmosphere, the sensor device comprising:
a measurement compartment (102) configured to receive a receptor element (209) of an electrochemical sensor for detecting one or more constituents of the test atmosphere;
a receptor support (104) arranged at an end of the measurement compartment and configured to support the receptor element of the electrochemical sensor; and
a transducer element (106) functionally couplable to the receptor element and configured to provide an electronically processable signal indicative of the one or more constituents in the test atmosphere.

2. The sensor device according to claim 1, wherein the measurement compartment defines an interior volume, in particular a cylindrical interior volume, of the sensor device configured to least partly encompass the receptor element of the electrochemical sensor along a perimeter.

3. The sensor device according to any one of the preceding claims, wherein the measurement compartment and the receptor support together form a cavity of the sensor device configured to hold a matrix (211) mimicking a surface of a cell culture, such as a cell culture medium, in particular a liquid and/or gelatinous cell culture medium, or another liquid, gelatinous, polymeric and/or porous material mimicking the surface of the cell culture.

4. The sensor device according to any one of the preceding claims, wherein the measurement compartment and/or the sensor device is sized, shaped and formed in correspondence with an interior volume of an exposure chamber and/or a well of a culture plate, in particular a 6-, 12- or 24-well cell culture plate, such that the receptor support and/or the receptor element of the electrochemical sensor is positionable at a predetermined position within the exposure chamber and/or within the well of the cell culture plate.

5. The sensor device according to any one of the preceding claims, wherein the receptor support is arranged and/or configured to position the receptor element relative to the transducer element, such that a functional contact between the transducer element and the receptor element of the electrochemical sensor is established.

6. The sensor device according to any one of the preceding claims, wherein at least a part of the transducer element is integrated into and/or embedded in the receptor support.

7. The sensor device according to any one of the preceding claims, wherein at least a part of the transducer element is attached to a side of the receptor support facing an interior volume defined by the measurement compartment.

8. The sensor device according to any one of the preceding claims, wherein the transducer element includes an array of electrodes (110) arranged at a side of the receptor support facing the measurement compartment, preferably wherein the array includes a working electrode, a counter electrode, and a reference electrode.

9. The sensor device according to any one of the preceding claims, further comprising at least one receptor element of an electrochemical sensor configured to physically and/or chemically interact with the one or more constituents of the test atmosphere.

10. The sensor device according to any one of the preceding claims, wherein the receptor element includes at least one of a molecular imprinted polymer, an antibody, a receptor protein, and an enzyme configured to interact with the one or more constituents in the test atmosphere, preferably wherein the receptor element includes a layer of at least one of a molecular imprinted polymer, an antibody, a receptor protein, and an enzyme configured to interact with the one or more constituents in the test atmosphere.

11. The sensor device according to any one of the preceding claims, further comprising:
one or more electrical leads (112) contacting the transducer element for power supply and/or for transmission of the electronically processible signal to a processing device.

12. The sensor device according to any one of the preceding claims, further comprising:
a holder configured to receive at least a part of the measurement compartment.

13. The sensor device according to claim 12, wherein the holder includes an interface configured to establish an electronic connection with the transducer element upon receipt of the measurement compartment in the holder.

14. The sensor device according to any one of the preceding claims, further comprising a processing device configured to process the electronically processible signal provided by the transducer element into a measure correlating with a dose associated with the one or more constituents of the test atmosphere.

15. Use of a sensor device according to any one of the preceding claims for determining the exposure of a cell culture to a test atmosphere, in particular for dose assessment.

## Patentansprüche

1. Sensorvorrichtung (100) für ein Aerosolexpositionssystem zur In-vitro-Exposition einer Zellkultur gegenüber einer Testatmosphäre, wobei die Sensorvorrichtung Folgendes umfasst:
eine Messkammer (102), die konfiguriert ist, ein Rezeptorelement (209) eines elektrochemischen Sensors zum Detektieren eines oder mehrerer Bestandteile der Testatmosphäre aufzunehmen;
einen Rezeptorträger (104), der an einem Ende der Messkammer angeordnet ist und konfiguriert ist, das Rezeptorelement des elektrochemischen Sensors zu tragen; und
ein Wandlerelement (106), das funktionell mit dem Rezeptorelement koppelbar ist und konfiguriert ist, ein elektronisch verarbeitbares Signal bereitzustellen, das den einen oder die mehreren Bestandteile in der Testatmosphäre anzeigt.

2. Sensorvorrichtung nach Anspruch 1, wobei die Messkammer ein Innenvolumen, insbesondere ein zylindrisches Innenvolumen der Sensorvorrichtung definiert, das so konfiguriert ist, dass es das Rezeptorelement des elektrochemischen Sensors entlang eines Umfangs zumindest teilweise umgibt.

3. Sensorvorrichtung nach einem der vorhergehenden Ansprüche, wobei die Messkammer und der Rezeptorträger zusammen einen Hohlraum der Sensorvorrichtung bilden, der konfiguriert ist, eine Matrix (211) aufzunehmen, die eine Oberfläche einer Zellkultur nachahmt, wie beispielsweise ein Zellkulturmedium, insbesondere ein flüssiges und/oder gelatineartiges Zellkulturmedium, oder ein anderes flüssiges, gelatineartiges, polymeres und/oder poröses Material, das die Oberfläche der Zellkultur nachahmt.

4. Sensorvorrichtung nach einem der vorhergehenden Ansprüche, wobei die Messkammer und/oder die Sensorvorrichtung in Übereinstimmung mit einem Innenvolumen einer Expositionskammer und/oder eines Napfes einer Kulturplatte, insbesondere einer 6-, 12- oder 24-Napf-Zellkulturplatte, bemessen, geformt und gebildet sind, sodass der Rezeptorträger und/oder das Rezeptorelement des elektrochemischen Sensors an einer vorbestimmten Position innerhalb der Expositionskammer und/oder innerhalb des Napfes der Zellkulturplatte positionierbar ist.

5. Sensorvorrichtung nach einem der vorhergehenden Ansprüche, wobei der Rezeptorträger so angeordnet und/oder konfiguriert ist, dass er das Rezeptorelement relativ zu dem Wandlerelement positioniert, sodass ein Funktionskontakt zwischen dem Wandlerelement und dem Rezeptorelement des elektrochemischen Sensors hergestellt wird.

6. Sensorvorrichtung nach einem der vorhergehenden Ansprüche, wobei zumindest ein Teil des Wandlerelements in dem Rezeptorträger integriert und/oder eingebettet ist.

7. Sensorvorrichtung nach einem der vorhergehenden Ansprüche, wobei zumindest ein Teil des Wandlerelements an einer Seite des Rezeptorträgers befestigt ist, die einem durch die Messkammer definierten Innenvolumen zugewandt ist.

8. Sensorvorrichtung nach einem der vorhergehenden Ansprüche, wobei das Wandlerelement eine Anordnung von Elektroden (110), die an einer Seite des Rezeptorträgers angeordnet sind, die der Messkammer zugewandt ist, umfasst, wobei die Anordnung bevorzugt eine Arbeitselektrode, eine Gegenelektrode und eine Referenzelektrode beinhaltet.

9. Sensorvorrichtung nach einem der vorhergehenden Ansprüche, die ferner mindestens ein Rezeptorelement eines elektrochemischen Sensors umfasst, das so konfiguriert ist, dass es physikalisch und/oder chemisch mit dem einen oder den mehreren Bestandteilen der Testatmosphäre wechselwirkt.

10. Sensorvorrichtung nach einem der vorhergehenden Ansprüche, wobei das Rezeptorelement mindestens eines von einem molekularen geprägten Polymer, einem Antikörper, einem Rezeptorprotein und einem Enzym beinhaltet, die so konfiguriert sind, dass sie mit dem einen oder den mehreren Bestandteilen in der Testatmosphäre wechselwirken, wobei das Rezeptorelement bevorzugt eine Schicht aus mindestens einem von einem molekularen geprägten Polymer, einem Antikörper, einem Rezeptorprotein und einem Enzym, die so konfiguriert sind, dass sie mit dem einen oder den mehreren Bestandteilen in der Testatmosphäre wechselwirken, beinhaltet.

11. Sensorvorrichtung nach einem der vorhergehenden Ansprüche, ferner umfassend:
eine oder mehrere elektrische Leitungen (112), die das Wandlerelement zur Energieversorgung und/oder zur Übertragung des elektronisch verarbeitbaren Signals an eine Verarbeitungsvorrichtung kontaktieren.

12. Sensorvorrichtung nach einem der vorhergehenden Ansprüche, ferner umfassend:
einen Halter, der konfiguriert ist, zumindest einen Teil der Messkammer aufzunehmen.

13. Sensorvorrichtung nach Anspruch 12, wobei der Halter eine Schnittstelle umfasst, die konfiguriert ist, bei Aufnahme der Messkammer in dem Halter eine elektronische Verbindung mit dem Wandlerelement herzustellen.

14. Sensorvorrichtung nach einem der vorhergehenden Ansprüche, die ferner eine Verarbeitungsvorrichtung umfasst, die konfiguriert ist, das elektronisch verarbeitbare Signal, das von dem Wandlerelement bereitgestellt wird, zu einem Messwert zu verarbeiten, der mit einer Dosis im Zusammenhang mit dem einen oder den mehreren Bestandteilen der Testatmosphäre korreliert.

15. Gebrauch einer Sensorvorrichtung nach einem der vorhergehenden Ansprüche zur Bestimmung der Exposition einer Zellkultur gegenüber einer Testatmosphäre, insbesondere zur Dosisbewertung.

## Revendications

1. Dispositif de capteur (100) pour un système d'exposition aux aérosols pour une exposition in vitro d'une culture cellulaire à une atmosphère d'essai, le dispositif de capteur comprenant :
un compartiment de mesure (102) configuré pour recevoir un élément récepteur (209) d'un capteur électrochimique pour détecter un ou plusieurs constituants de l'atmosphère d'essai ;
un support de récepteur (104) agencé au niveau d'une extrémité du compartiment de mesure et configuré pour supporter l'élément récepteur du capteur électrochimique ; et
un élément transducteur (106) pouvant être couplé fonctionnellement à l'élément récepteur et configuré pour fournir un signal pouvant être traité électroniquement indicatif des un ou plusieurs constituants dans l'atmosphère d'essai.

2. Dispositif de capteur selon la revendication 1, dans lequel le compartiment de mesure définit un volume intérieur, en particulier un volume intérieur cylindrique, du dispositif de capteur configuré pour entourer au moins partiellement l'élément récepteur du capteur électrochimique le long d'un périmètre.

3. Dispositif de capteur selon l'une quelconque des revendications précédentes, dans lequel le compartiment de mesure et le support de récepteur forment ensemble une cavité du dispositif de capteur configurée pour maintenir une matrice (211) imitant une surface d'une culture cellulaire, telle qu'un milieu de culture cellulaire, en particulier un milieu de culture cellulaire liquide et/ou gélatineux, ou un autre matériau liquide, gélatineux, polymère et/ou poreux imitant la surface de la culture cellulaire.

4. Dispositif de capteur selon l'une quelconque des revendications précédentes, dans lequel le compartiment de mesure et/ou le dispositif de capteur est dimensionné, façonné et formé en correspondance avec un volume intérieur d'une chambre d'exposition et/ou d'un puits d'une plaque de culture, en particulier une plaque de culture cellulaire à 6, 12 ou 24 puits, de telle sorte que le support de récepteur et/ou l'élément récepteur du capteur électrochimique peut être positionné au niveau d'une position prédéterminée au sein de la chambre d'exposition et/ou au sein du puits de la plaque de culture cellulaire.

5. Dispositif de capteur selon l'une quelconque des revendications précédentes, dans lequel le support de récepteur est agencé et/ou configuré pour positionner l'élément récepteur par rapport à l'élément transducteur, de telle sorte qu'un contact fonctionnel entre l'élément transducteur et l'élément récepteur du capteur électrochimique est établi.

6. Dispositif de capteur selon l'une quelconque des revendications précédentes, dans lequel au moins une partie de l'élément transducteur est intégrée et/ou noyée dans le support de récepteur.

7. Dispositif de capteur selon l'une quelconque des revendications précédentes, dans lequel au moins une partie de l'élément transducteur est fixée à un côté du support de récepteur faisant face à un volume intérieur défini par le compartiment de mesure.

8. Dispositif de capteur selon l'une quelconque des revendications précédentes, dans lequel l'élément transducteur comporte un réseau d'électrodes (110) agencé sur un côté du support de récepteur faisant face au compartiment de mesure, de préférence dans lequel le réseau comporte une électrode de travail, une contre-électrode et une électrode de référence.

9. Dispositif de capteur selon l'une quelconque des revendications précédentes, comprenant en outre au moins un élément récepteur d'un capteur électrochimique configuré pour interagir physiquement et/ou chimiquement avec les un ou plusieurs constituants de l'atmosphère d'essai.

10. Dispositif de capteur selon l'une quelconque des revendications précédentes, dans lequel l'élément récepteur comporte au moins l'un parmi un polymère à empreinte moléculaire, un anticorps, une protéine récepteur et une enzyme, configurés pour interagir avec les un ou plusieurs constituants dans l'atmosphère d'essai, de préférence dans lequel l'élément récepteur comporte une couche d'au moins l'un parmi un polymère à empreinte moléculaire, un anticorps, une protéine récepteur et une enzyme, configurés pour interagir avec les un ou plusieurs constituants dans l'atmosphère d'essai.

11. Dispositif de capteur selon l'une quelconque des revendications précédentes, comprenant en outre :
un ou plusieurs conducteurs électriques (112) en contact avec l'élément transducteur pour l'alimentation électrique et/ou pour la transmission du signal pouvant être traité électroniquement à un dispositif de traitement.

12. Dispositif de capteur selon l'une quelconque des revendications précédentes, comprenant en outre :
un portoir configuré pour recevoir au moins une partie du compartiment de mesure.

13. Dispositif de capteur selon la revendication 12, dans lequel le portoir comporte une interface configurée pour établir un raccordement électronique avec l'élément transducteur lors de la réception du compartiment de mesure dans le portoir.

14. Dispositif de capteur selon l'une quelconque des revendications précédentes, comprenant en outre un dispositif de traitement configuré pour traiter le signal pouvant être traité électroniquement fourni par l'élément transducteur en une mesure en corrélation avec une dose associée aux un ou plusieurs constituants de l'atmosphère d'essai.

15. Utilisation d'un dispositif de capteur selon l'une quelconque des revendications précédentes pour déterminer l'exposition d'une culture cellulaire à une atmosphère d'essai, en particulier pour l'évaluation de la dose.
